# EUROPEAN PATENT APPLICATION

(11) **EP 4 159 853 A1**
(43) Date of publication of application: **05.04.2023**
(21) Application number: 20938166.4
(22) Date of filing: 30.11.2020
(51) Int. Cl.: C12N 9/22, C12N 15/52, C12N 15/113, C07K 14/00

(54) **GENOME EDITING SYSTEM AND METHOD**

(30) Priority: 28.05.2020 WO PCT/CN2020/092799
(71) Applicant: ShanghaiTech University, Shanghai 201210 (CN)
(72) Inventor: JI, Quanjiang, Shanghai 201210 (CN); WU, Zhaowei, Shanghai 201210 (CN); ZHANG, Yifei, Shanghai 201210 (CN); WANG, Yujue, Shanghai 201210 (CN); WANG, Yannan, Shanghai 201210 (CN)
(74) Representative: Dehns
(86) International application number: PCT/CN2020/132759
(87) International publication number: WO 2021/238128

(57) **Abstract**

Provided is a genome editing system and method for gene editing at least one target sequence in a cell genome. The genome editing system comprises: (1) an expression construct comprising a Cas12f nuclease; and (2) an expression DNA sequence comprising a guide RNA corresponding to the Cas12f nuclease, and an expression construct of the targeting sequence of the target sequence. The gene editing system or method can accurately knock out a target gene from within a cell; in addition, in an *in vitro* cutting experiment, the target gene can be accurately cut.

## Description

This application claims the priority of PCT international patent application PCT/CN2020/092799 filed on May 28, 2020, the contents of which are incorporated herein by its entirety.

### TECHNICAL FIELD

The present disclosure relates to the field of biotechnology, in particular to a genome editing system and method, specifically to a novel genome editing method based on a very small CRISPR/Cas12f nuclease.

### BACKGROUND

Genome editing technology refers to a genetic engineering technique that uses gene editing tools, such as programmable nucleases (molecular scissor), to cut off specific gene sequences and then introduce gene insertions, deletions or substitutions to achieve modification on specific genomic DNA fragments of an organism, thereby achieving the editing of the target gene. The use of genome editing technology for genetic manipulation of cells or species can be widely used in basic research of life science, medicine, agronomy and other fields, biotechnology development, and disease treatment development and research. For example, correction of mutated genes in diseases such as genetic disorders or cancer; genetic engineering of crops to enhance their performance; precise modification of microbial genomes to promote the production of high added value compounds; cleaving pathogenic microbial genomes to kill pathogenic microorganisms for infection treatment, etc.

Since its introduction, the CRISPR/Cas (Clustered regularly interspaced short palindromic repeats/CRISPR-associated protein) genome editing system has been widely used in biology, medicine, agronomy and other fields due to its simplicity and efficiency. Cas nucleases use guide RNAs to target genome-specific sites in a variety of cells, cleave the genome-specific sites to create DNA double-strand breaks, and then using endogenous or exogenous DNA repairing mechanisms, such as homologous recombination and non-homologous recombination end-joining to edit. Depending on the activation of different DNA repairing pathways, genome editing will result in inactivation of genes or correction of mutations. By fusing inactivated Cas nucleases with base deaminase or reverse transcriptase, the CRISPR/Cas system can be further modified into base editing systems and Prime editing systems for a wide range of applications in biology, agronomy research, and disease treatment.

Currently, the widely used CRISPR/Cas genome editing systems mainly include two types as CRISPR/Cas9 and CRISPR/Cas12a. In both types of genome editing systems, the CRISPR effector protein nucleases Cas9 and Cas12a are large proteins that contain more than 1000 amino acids, resulting in significant problems with the delivery of CRISPR/Cas9 or CRISPR/Cas12a to cells. For example, CRISPR/Cas9 or CRISPR/Cas12a systems often need to be packaged into adeno-associated virus vectors (AAVs) for use in gene therapy in a living body. The large molecular size of CRISPR/Cas9 and CRISPR/Cas12a systems greatly limits the packaging efficiency of adeno-associated virus vectors, increases the packaging difficulty, and greatly limits the use of these systems in a wide range of applications such as gene therapy. Therefore, it is urgent to discover and develop efficient genome editing systems with a small molecular weight.

### SUMMARY

The technical problem to be solved in the present disclosure is for overcoming the lack of small nucleases for gene editing in the art, and provided is a gene editing system and method; in particular a novel genome editing method based on a miniscule CRISPR/Cas12f nuclease. It is possible to perform precise knockout or precise editing on target genes in cells by using the gene editing system or method of the present disclosure; and to cut off the target gene sequences precisely in cleavage experiments *in vitro.*

The present disclosure solves the above-mentioned technical problems through the following technical solutions.

The first technical solution of the present disclosure is: a genome editing system for gene editing at least one target sequence in a living cell genome, wherein the genome editing system comprising:
(1) an expression construct comprising a Cas12f nuclease;
(2) an expression construct comprising an expression DNA sequence of a guide RNA corresponding to the Cas12f nuclease, and a targeting sequence of the target sequence.

Herein, the guide RNA is preferably set forth in a sequence of SEQ ID NO: 3 in sequence listing.

The targeting sequence is preferably a DNA fragment with a length of 20 bp downstream of PAM sequence.

The Cas12f nuclease in the present disclosure can be a Cas12f nuclease conventional in the art, preferably has a source selected from a group consisting of: *Syntrophomonas palmitatica* Cas12f (SpCas12f1), *Acidibacillus sulfuroxidans* Cas12f

(AsCas12f1), *Eubacterium siraeum* Cas12f (EsCas12f1) and *Clostridium novyi* Cas12f (CnCas12f1).

In a preferred embodiment of the present disclosure, the Cas12f nuclease is set forth in an amino acid sequence of SEQ ID NO: 1 in sequence listing; preferably, the Cas12f nuclease is set forth in a coding sequence of SEQ ID NO: 2 in sequence listing.

The second technical solution of the present disclosure is: a genome editing method comprising introducing the genome editing system as described above into a cell comprising a target sequence to perform genome editing.

The "cell" described in the present disclosure can be bacteria, such as *Escherichia coli, Klebsiella pneumoniae*; it can also be a mammalian cell.

The "gene editing" in the genome editing method as described above can be conventional in the art, such as, but not limited to, gene cleavage, gene deletion, gene insertion, point mutation, transcription inhibition, transcription activation and base editing, etc.; more preferably, gene deletion or gene cleavage.

The third technical solution of the present disclosure is: a cleavage method of double strand DNA comprising mixing Cas12f nuclease, complete guide RNA and the double strand DNA; wherein the complete guide RNAis: 5'- guide RNA corresponding to the Cas12f nuclease + targeting sequence of the double strand DNA-3'.

As described above, the guide RNA corresponding to the Cas12f nuclease is preferably shown in SEQ ID NO: 3 of sequence listing. The complete guide RNA can be formed by adding a targeting sequence of 20 bases of different genes at the 3' terminus.

The preferred definitions of the Cas12f nuclease are as described above, preferably the Cas12f nuclease has a source selected from a group consisting of: *Syntrophomonas palmitatica* Cas12f (SpCas12f1), *Acidibacillus sulfuroxidans* Cas12f (AsCas12f1), *Eubacterium siraeum* Cas12f (EsCas12f1) and *Clostridium novyi* Cas12f (CnCas12f1); more preferably the Cas12f nuclease is shown in SEQ ID NO: 1 or a variant thereof of sequence listing; further more preferably, the Cas12f nuclease is set forth in a coding sequence of SEQ ID NO: 2 in sequence listing. The variant has at least 20% (e.g., 30%, 40%, 50%, 60%, 70%, 80%, 85%, 90%, 95%, 99%) sequence identity with the amino acid residues of SEQ ID NO: 1 and retains double strand DNA cleavage activity.

The cleavage method of the present disclosure can be conventional in the art, for example, in a preferred embodiment of the present disclosure: the cleavage method is performed in a pH7.5 buffer solution containing 25 mM NaCl, 5 mM MgCl₂, and 10 mM Tris-HCl.

The amount of the Cas12f nuclease in the present disclosure can refer to other Cas nucleases that used in the art for reactions, for example, in a preferred embodiment of the present disclosure: the mole ratio of the Cas12f nuclease to the complete guide RNA is 1:2.

The reaction temperature of the cleavage method is preferably 37°C and the reaction time is preferably 30 min.

The targeting sequence is preferably a DNA fragment with a length of 20 bp downstream of PAM sequence.

The fourth technical solution of the present disclosure is: a use of Cas12f nuclease in gene editing.

Further definitions of the Cas12f nuclease and the gene editing are as described above.

The size of the nuclease of the gene editing system does not exceed 700 amino acids.

Positive progressive effects of the present disclosure:
The present disclosure shows that Cas12f is able to cleave genomic DNA precisely and achieve genomic DNA double-strand breaks through the guidance and localization functions of the guide RNA. By using the intrinsic or exogenous repair mechanisms of the host cell, the system is able to achieve gene editing efficiently and precisely in living cells.

### BRIEF DESCRIPTION OF THE DRAWINGS

Figure 1 is a schematic diagram of the CRISPR-SpCas12f1-based cellular genome editing method.

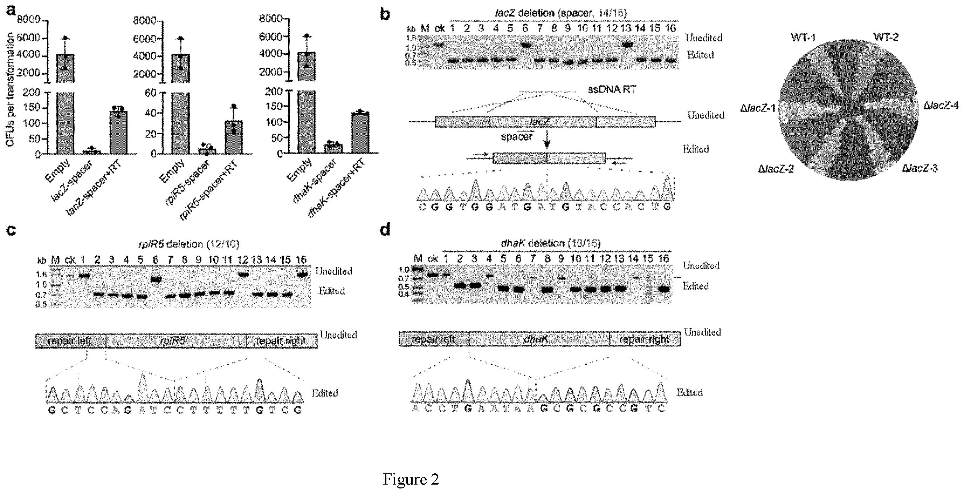

Figure 2 shows CRISPR-SpCas12f1 for efficient genome editing in living cells; Figure 2a: CRISPR-SpCas12f1 targets and cleaves the bacterial genome efficiently to achieve bacterial death. The lacZ-spacer, rpiR5-spacer, and dhaK-spacer refer to the pgRNA plasmids ligated into the targeted gene, respectively, and RT refers to the single strand DNA repair template with a length of 90 nt. Figure 2b: CRISPR-SpCas12f1 achieves efficient lacZ gene deletion. The lacZ gene-deleted strains lost the ability to produce blue color in the blue-white experiments. Figure 2c: CRISPR-SpCas12f1 achieves efficient rpiR5 gene deletion. Figure 2d: CRISPR-SpCas12f1 achieves efficient dhaK gene deletion.
Figure 3 shows the efficient double strand DNA cleavage achieved by CRISPR-SpCas12f1. The samples in the empty lane only contained the DNA to be cleaved, while the samples in lanes 1 and 2 were added with the DNA to be cleaved, and SpCas12f1 nuclease and guide RNA, respectively. The sample in lane 1 was reacted at 37°C for 10 min, while the sample in lane 2 was reacted at 37°C for 30 min.
Figure 4 is the flow and results of the identification of the protospacer adjacent motif (PAM) of AsCas12f1. The results show that AsCas12f1 can recognize PAM sequences of 5'-TTR type effectively (wherein R is a degenerate base, representing A or G).
Figure 5 is the results of cleavage of substrate DNA by AsCas12f1 nuclease. The results show that the substrate DNA was cut into two DNA fragments by AsCas12f1 precisely. And its cleavage activity depends on the recognition of PAM sequence, the regularity of which is fully consistent with the protospacer adjacent motif (PAM) obtained from the identification.

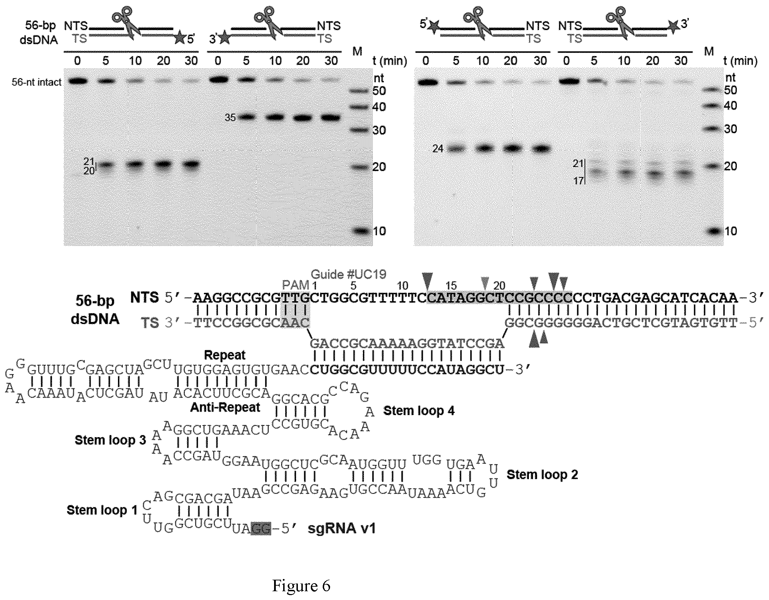

Figure 6 is the results of cleavage of four fluorescence labeled DNA substrates with a length of 56 bp with AsCas12f1 nuclease. The results show that the cleavage site of AsCas12f1 in the targeting strand is 3-4 nt downstream the sequence complementary to spacer, while the cleavage site in the non-targeting strand is between positions 12 and 13 in the protospacer. After the specific cleavage of the non-targeting strand, AsCas12f1 can also non-specifically cleave the single strand DNA of the non-targeting strand replaced by the binding of sgRNA, with the cleavage length concentrated at about 13 nt. The resulting cleavage product has a sticky protruding end with a length of 11 nt at 5' terminus, and a sticky protruding end with a length of 0-4 nt at 3' terminus.

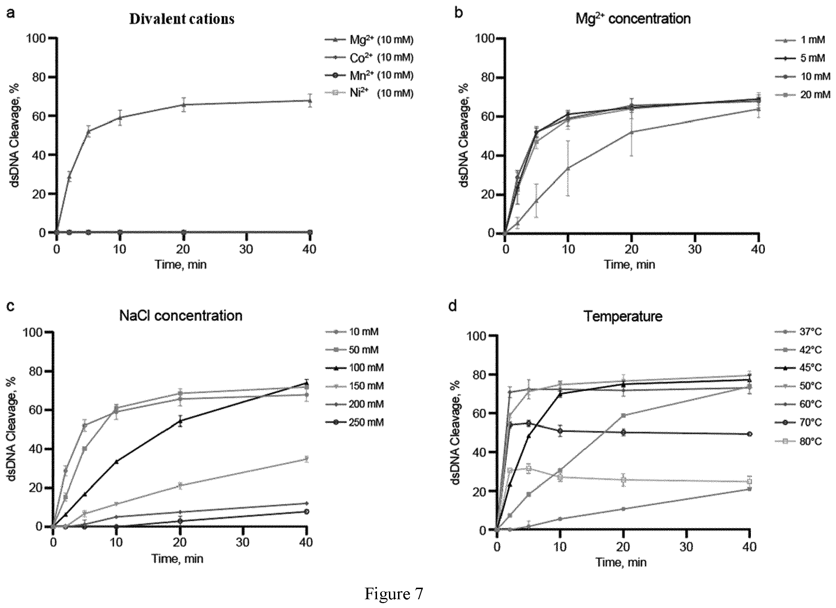

Figure 7a and Figure 7b are the results of biases of AsCas12f1 nuclease for the type and concentration of divalent cation. The results show that the activity of AsCas12f1 depends on Mg²⁺, and the concentration of Mg²⁺ should be 5 mM or more. Figure 7c is the results of the bias of AsCas12f1 nuclease for NaCl concentration. The results show that AsCas12f1 is relatively sensitive to NaCl concentration, and the concentration of NaCl should be 100 mM or less. Figure 7d is the results of the temperature bias of AsCas12f1 nuclease. The results show that the optimum temperature for cleavage of DNA by AsCas12f1 between 45-60 centigrade.
Figure 8a and Figure 8b are the results of the biases of AsCas12f1 nuclease for spacer length. The results show that the optimal spacer length of AsCas12f1 between 17-20 nt.
Figure 9a is the results of structural simulation of AsCas12f1. Figure 9b is the comparison results of double strand DNA cleavage activity of four mutants and wild-type AsCas12f1 protein. The results show that all four mutants can inactivate the cleavage ability ofAsCas12f1 on targeting strand, while mutants R383A and D401A still retain weak cleavage activity (nickase activity) on the non-targeting strand.
Figure 10 shows the method and results of gene editing in living cells of *E*. *coli* and *K. pneumoniae.* The method is shown in Figure 10a. The hisD gene in *E*. *coli* and the pyrF gene in *K. pneumoniae* were selected for the experiments, respectively. Figure 10b is the results of gene editing in *E. coli* using AsCas12f1 nuclease. Figure 10c is the results of gene editing in *K. pneumoniae* using AsCas12f1 nuclease. The results show that the hisD gene in *E. coli* and the pyrF gene in *K. pneumoniae* can be precisely edited by AsCas12f1 nuclease.
Figure 11 is the results of AsCas12f1 transient expression plasmid-mediated gene editing in human cells. The results show that AsCas12f1 nuclease successfully achieved efficient gene editing of seven sites on genes VEGFA, TP53, HEXA, PRNP and PDCD1.
Figure 12 is the statistical results of high-throughput sequencing after AsCas12f1 transient expression plasmid-mediated gene editing in human cells. As shown in the figure, AsCas12f1 nuclease can introduce insertion or deletion mutations at the target sequence precisely.
Figure 13 is the results of AsCas12f1 ribonucleoprotein complex-mediated gene editing in human cells. As shown in the figure, AsCas12f1 nuclease successfully achieved efficient gene editing of six sites on genes VEGFA, TP53, HEXA and PDCD1.
Figure 14 is the results of SpCas12f1 transient expression plasmid-mediated gene editing in human cells. As shown in the figure, AsCas12f1 nuclease successfully achieved efficient gene editing of four sites on gene VEGFA.
Figure 15 is the results of cleavage of substrate DNA by EsCas12f1 nuclease. The results show that the substrate DNA was cut into two DNA fragments by EsCas12f1 precisely, and EsCas12f1 preferred the PAM sequence of 5'-TTH type (wherein H stands for A, C and T).

### DETAILED DESCRIPTION OF EMBODIMENTS

The present disclosure is further illustrated combining with specific examples below. It should be understood that these examples are only intended to illustrate the present disclosure and are not intended to limit the scope of the present disclosure. Moreover, it should be understood that after reading the content of the present disclosure, a person skilled in the art may make various alterations or modifications to the present disclosure, and such equivalents may also fall within the scope of the appended claims of the present application. For example, developing a Cas12f-based single-base editing system by fusing inactivated Cas12f and base deaminase; developing a Cas12f-based Prime editing system by fusing inactivated Cas12f and reverse transcriptase; developing a Cas12f-based transcription activation system by fusing inactivated Cas12f and transcription activation factor; developing a Cas12f-based epigenetic modification system by fusing inactivated Cas12f and nucleic acid epigenetic modification enzyme; and developing a Cas12f-based transcription inhibition system by using inactivated Cas12f.

It should be noted that the terms "an" or "a" entity in the present disclosure refers to one or more such entities; thus, the terms "an" (or "a"), "one or more" and "at least one" are used interchangeably herein.

The term "homology" or "identity" or "similarity" refers to the sequence similarity between two peptides or between two nucleic acid molecules. Homology can be determined by comparing corresponding positions in different polypeptides or nucleic acid molecules, and when the same position in a sequence of the molecule being compared is occupied by the base or amino acid same to that in a different sequence, the molecule is homologous at that position. The degree of homology between sequences is determined by the function of the number of common matching or homologous positions in the sequences. An "irrelavant" or a "non-homologous" sequence should have a homology of less than 20% to one of the sequences disclosed in the present disclosure.

A certain percentage of sequence homology (e. g ., 20%, 30%, 40%, 50%, 60%, 70%, 80%, 90%, 95%, 98% or 99%) between a polynucleotide or a polynucleotide region (or a polypeptide or a polypeptide region) and another polynucleotide acid or polynucleotide region (or a polypeptide or a polypeptide region) means that such percentage of bases (or amino acids) in the two sequences aligned are the same upon alignment. This alignment and homology (%) or sequence identity can be determined using software programs and methods known in the art, such as those described in Ausubel et al. eds . (2007) Current Protocols in Molecular Biology*.* Preferably, default parameters should be used for sequence alignment. One alternative alignment program is BLAST, using default parameters. In particular, when using the programs BLASTN and BLASTP for alignment, the following default parameters are used: Genetic code=standard; filter=none; strand=both; cutoff=60; expect= 10; Matrix = BLOSUM62; Descriptions = 50sequences; sort by = HIGH SCORE; Databases = non-redundant, GenBank+EMBL+DDBJ+PDB+GenBank CDS translations+SwissProtein+SPupdate+PIR. Polynucleotides that are considered biologically equivalent refer to those polynucleotides that have the defined homology (%) described above and that encode polypeptides having the same or similar biological activity.

When the polynucleotide is DNA, the sequence of the polynucleotide consists of the letters representing the following four nucleotide bases: adenine (A), cytosine (C), guanine (G), and thymine (T). When the polynucleotide is RNA, the sequence of the polynucleotide consists of letters representing the following four nucleotide bases: adenine (A), cytosine (C), guanine (G), and uracil (U). Thus, the term "polynucleotide sequence" is an alphabetic representation of a polynucleotide molecule. This alphabetic representation can be put into a database in a computer with a central processing unit and used for bioinformatics applications, such as functional genomics and homology searches. The term "polymorphism" refers to the coexistence of more than one form of a gene or part thereof, and the term "polymorphic region of a gene" refers to a gene having different nucleotide presentations at the same position (i.e., different nucleotide sequences). Polymorphic region of a gene can be a single nucleotide, which is different in different alleles.

In the present disclosure, the terms "polynucleotide" and "oligonucleotide" can be used interchangeably, and they refer to any length of polymeric forms of nucleotides, whether deoxyribonucleotides or ribonucleotides or analogues thereof. Polynucleotides can have any three-dimensional structure and can perform any function known or unknown. Examples of polynucleotides include, but are not limited to, the following: genes or gene fragments (including probes, primers, EST or SAGE tags), exons, introns, messenger RNA (mRNA), transfer RNA, ribosomal RNA, nucleases, cDNA, dsRNA, siRNA, miRNA, recombinant polynucleotides, branching polynucleotides, plasmids, vectors, isolated DNA of any sequence, isolated RNA of any sequence, nucleic acid probes and primers. Polynucleotides also comprise modified nucleotides, such as methylated nucleotides and nucleotide analogs. If a modification is present on the polynucleotide, the modification may be given before or after assembly of the polynucleotide. The nucleotide sequence can be interrupted by non-nucleotide components. The polynucleotide may be further modified after polymerization, such as being labeled by coupling to a labeled component. This term refers to both double strand and single strand polynucleotide molecules. Unless otherwise stated or claimed, any embodiment of a polynucleotide disclosed by the present disclosure includes either of its double strand form or any one of the two complementary single strand forms known or predicted to constitute the double strand form.

When applied to a polynucleotide, the term "encoding" means that the polynucleotide "encodes" a polypeptide, meaning that in its natural state or when manipulated by methods known to a person skilled in the art, it can be transcribed and/or translated to produce a target polypeptide and/or fragment thereof, or an mRNA capable of encoding that target polypeptide and/or fragment thereof. Antisense strand means a sequence that is complementary to the polynucleotide and from which the coding sequence can be deduced.

### Cas12f nuclease

Cas12f nuclease is able to be located to a target gene precisely under the guidance of the corresponding guide RNA to cleave genomic DNA, resulting a genomic DNA double strand break.

Although Cas12f has only half or less of the molecular weight than Cas9 and Cas12a, Cas12f has similar or higher genome cleavage efficiency than Cas9 and Cas12a, enabling efficient genome editing, leading to a great application prospect in fields such as gene therapy.

Accordingly, in the examples of the present disclosure, a novel genome editing method based on a miniscule CRISPR-Cas12f nuclease is disclosed. The present disclosure shows that Cas12f is able to cleave genomic DNA precisely through the guidance and localization function of a guide RNA and achieve genomic DNA double-strand breaks. Using the host cell's own or exogenous repair mechanisms, the system is able to achieve gene editing efficiently and precisely in living cells.

In some examples, the Cas12f nuclease of the gene editing system is derived from the group comprising the following: *Syntrophomonas palmitatica* Cas12f (SpCas12f1), *Acidibacillus sulfuroxidans* Cas12f (AsCas12f1), *Eubacterium siraeum* Cas12f (EsCas12f1) and *Clostridium novyi* Cas12f (CnCas12f1).

The following is a clear and complete description of the embodiments of the present disclosure in combination with the examples. Obviously, the examples described are only intended to illustrate a portion of the examples of the present disclosure and should not be considered as limiting the scope of the present disclosure. Specific conditions not specified in the examples, shall be carried out according to the conventional conditions or the conditions suggested by the manufacturer. Reagents or instruments not specified by the manufacturer are considered to be routine and commercially available.

The sources of biomaterials used in each example were as follows:
pSGKP plasmid (purchased from Addgene, USA, Cat no. 117233), pCasKP plasmid (purchased from Addgene, USA, Cat no. 117231), *Klebsiella pneumoniae* strain NCTC9633 (purchased from ATCC, USA, Cat no. 13883), competent *Escherichia coli* DH5alpha strain (purchased from CoWin Biotech Co., Ltd., Cat no. CW0808S). In each example, the LB liquid medium was purchased from Sangon Biotech (Shanghai) Co., Ltd., Cat no. A507002-0250; the LB solid medium was purchased from Sangon Biotech (Shanghai) Co., Ltd., Cat no. A507003-0250; Apramycin was purchased from BioVision, USA, Cat no. B1521-1G; Kanamycin was purchased from Shanghai Aladdin Bio-Chem Technology Co., Ltd., Cat no. K103024-5g.

### Example 1 Construction of expression plasmid pCas12f of SpCas12f1

The sequence of pCas12f is shown in SEQ ID NO: 4, and the detailed construction method thereof was as follows:
The pCasKP plasmid was used as a template to amplify a fragment containing the λ-Red recombination system and Apramycin resistance gene. The p15a replicon sequence was used as a template to amplify the p15a replicon, wherein the p15a replicon sequence was synthesized by Sangon Biotech (Shanghai) Co., Ltd., and is shown in SEQ ID NO: 5. The SpCas12f1 gene sequence was used as a template to amplify the SpCas12f1 gene, wherein the SpCas12f1 gene sequence was synthesized by Sangon Biotech (Shanghai) Co., Ltd. , and is shown in SEQ ID NO: 1.
5' primer sequence for amplifying the fragment containing λ-Red recombination system and Apramycin resistance gene (SEQ ID NO: 6):
   5'-TACGATATAAGTTGTGACGTaaaatgagacgttgatcggc-3'
3' primer sequence for amplifying the fragment containing λ-Red recombination system and Apramycin resistance gene (SEQ ID NO: 7):
   5'-taaatagctcctggttttagcttttg-3'
5' primer sequence for amplifying p15a replicon (SEQ ID NO: 8):
   5'-ttcactgatagatacaagagCGTCGGGTGATGCTGCCAAC-3'
3' primer sequence for amplifying p15a replicon (SEQ ID NO: 9):
   5'-gccgatcaacgtctcattttACGTCACAACTTATATCGTATGGGG-3'
5' primer sequence for amplifying SpCas12f1 gene (SEQ ID NO: 10):
   5'-aaagctaaaaccaggagctatttaATGGGTGAAAGCGTTAAAGCGATC-3'
3' primer sequence for amplifying SpCas12f1 gene (SEQ ID NO: 11):

5’-CCGACGctcttgtatctatcagtgaagcatcaagactaacaaacaaaagtagaacaactgttcaccgt
tacatatcaaagggaaaactgtccatatgggtatttaccacaacagtacgccaaccagccaTTATTTAGCTTTAC
GGATCTTTTTCGGTTTC-3’

The mentioned above three DNA fragments were amplified respectively using PrimeSTAR HS DNA Polymerase of Takara, and the reaction system was: 34.4 µL ddH₂O, 4 µL dNTP Mixture (2.5 mM each), 10 µL 5×Primestar Buffer, 0.3 µL 5'-Primer (50 µM), 0.3 µL 3'-Primer (50 µM), 0.5 µL template DNA (100 ng/µL), 0.5 µL PrimerSTAR HS DNA Polymerase.

The mentioned above PCR reaction system was prepared, followed by polymerase chain reaction (PCR) with the following cycling conditions: 98°C for 30 s; then 98°C for 10 s, 55°C for 30 s and 72°C for 5 min under a total of 30 cycles; finally 72°C for 10 min. The PCR products were recovered respectively using the SanPrep column PCR product purification kit manufactured by Sangon Biotech (Shanghai) Co., Ltd. The specific steps of PCR product purification were carried out according to the kit operation manual.

The three DNA fragments obtained were assembled to one plasmid. The specific reaction system was: 10 µL of NEBuilder HiFi DNA Assembly Master Mix (NEB), 20 fmol of each DNA fragment, and appropriate amount of ddH₂O was added to a total volume of 20 µL. The reaction was carried out at 50°C for 1 h. 20 µL of the reaction product was transformed into *E. coli* competent DH5α strain and plated on a LB solid culture plate containing 50 µg/mL of Apramycin. After the transformation solution was absorbed by the solid culture plate, the plate was inverted and incubated overnight at 37°C in an incubator. The transformed strains grown on the plate were transferred and stored, and the plasmids were extracted for subsequent experiments using the SanPrep Column Plasmid DNA Small Volume Extraction Kit of Sangon Biotech (Shanghai) Co., Ltd. Meanwhile, the plasmids were sent to Sangon Biotech (Shanghai) Co., Ltd. for sequencing confirmation.

Example 2 Construction of expression plasmid pgRNA of the guide RNA corresponding to SpCas12f1

The sequence of pgRNA is shown in SEQ ID NO: 12, and the detailed construction method thereof was as follows:

The pSGKP plasmid was used as a template to amplify the plasmid backbone. The expression DNA sequence of the guide RNA corresponding to SpCas12f1 was used as a template to amplify the DNA sequence of the guide RNA. Herein the expression DNA sequence of the guide RNA corresponding to SpCas12f1 was synthesized by Sangon Biotech (Shanghai) Co., Ltd., and is shown in SEQ ID NO: 13. In addition to the DNA sequence of the guide RNA, SEQ ID NO: 13 also includes the promoter sequence and terminator sequence.
5' primer sequence for amplifying pSGKP plasmid backbone (SEQ ID NO: 14):
   5'- gatatcgaattcctgcagcccgg-3'
3' primer sequence for amplifying pSGKP plasmid backbone (SEQ ID NO: 15):
   5'-actagtattatacctaggactgagctagct-3'
5' primer sequence for amplifying the expression DNA sequence of the guide RNA (SEQ ID NO: 16):
   5' -ttgacagctagctcagtcctagg-3'
3' primer sequence for amplifying pSGKP plasmid backbone (SEQ ID NO: 17):
   5'-ccgggctgcaggaattcgatatc-3'

The mentioned above two DNA fragments were amplified respectively using PrimeSTAR HS DNA Polymerase of Takara, and the reaction system was: 34.4 µL ddH₂O, 4 µL dNTP Mixture (2.5 mM each), 10 µL 5×Primestar Buffer, 0.3 µL 5'-Primer (50 µM), 0.3 µL 3'-Primer (50 µM), 0.5 µL template DNA (100 ng/µL), 0.5 µL PrimerSTAR HS DNA Polymerase.

The mentioned above PCR reaction system was prepared, followed by polymerase chain reaction (PCR) with the following cycling conditions: 98°C for 30 s; then 98°C for 10 s, 55°C for 30 s and 72°C for 5 min under a total of 30 cycles; finally 72°C for 10 min. The PCR products were recovered respectively using the SanPrep column PCR product purification kit manufactured by Sangon Biotech (Shanghai) Co., Ltd. The specific steps of PCR product purification were carried out according to the kit operation manual.

The two DNA fragments obtained were assembled to one plasmid. The specific reaction system was: 10 µL of NEBuilder HiFi DNA Assembly Master Mix (NEB), 20 fmol of each DNA fragment, and appropriate amount of ddH₂O was added to a total volume of 20 µL. The reaction was carried out at 50°C for 1 h. 20 µL of the reaction product was transformed into *E. coli* competent DH5α strain and plated on a LB solid culture plate containing 50 µg/mL of Kanamycin. After the transformation solution was absorbed by the solid culture plate, the plate was inverted and incubated overnight at 37°C in an incubator. The transformed strains grown on the plate were transferred and stored, and the plasmids were extracted for subsequent experiments using the SanPrep Column Plasmid DNA Small Volume Extraction Kit of Sangon Biotech (Shanghai) Co., Ltd. Meanwhile, the plasmids were sent to Sangon Biotech (Shanghai) Co., Ltd. for sequencing confirmation.

### Example 3 Insertion of spacer fragment into plasmid pgRNA

Firstly, a DNA fragment with 20 bases (these 20 bases are called spacer, wherein TTC is not included) after a sequence of TTC (the PAM sequence recognized by SpCas12f1 in this example is TTC) was selected from the target gene of *Klebsiella pneumoniae.* Such step is characterized by the addition of aaac at the 5' terminus of this single strand DNA sequence, in order to enable the insertion of the spacer fragment to plasmid pgRNA. Meanwhile, a reverse complementary sequence to the spacer was synthesized with cgac being added at the 5' terminus of the reverse complementary sequence. For example, the selected DNA sequence (SEQ ID NO: 18) of the spacer of the lacZ gene in the experiment was: 5'- ATGGCGGTTAACGCCGCGGA-3', and the specific sequence design was as follows:
5'-aaacATGGCGGTTAACGCCGCGGA -3'(SEQ ID NO: 19)
5'-cgacTCCGCGGCGTTAACCGCCAT -3'(SEQ ID NO: 20)

The specific sequence design of dhaK was:
5'- aaacAGGCGGTGACCGGCGAGGCG-3'(SEQ ID NO: 21)
5'-cgacCGCCTCGCCGGTCACCGCCT-3'(SEQ ID NO: 22)

The specific sequence design of rpiR5 was:
5'-aaacGCGGTGACGACCCGCTGCGT-3'(SEQ ID NO: 23)
5'-cgacACGCAGCGGGTCGTCACCGC-3'(SEQ ID NO: 24)

The mentioned above two primers were synthesized with conventional method by Sangon Biotech (Shanghai) Co., Ltd.

Each pair of spacer primers designed and synthesized was phosphorylated with the following reaction system: 5 µL 10× T4 DNAligase Buffer (NEB), 2 µL spacer 5'-primer (50 µM), 2 µL spacer 3 '-primer (50 µM), 1 µL T4 polynucleotide kinase (Takara), and 40 µL ddH₂O. The reaction was carried out at 37°C for 1 h. 2.5 µL of 1 M NaCl was added into the reaction product and heated at 95°C for 5 min, then the temperature of the reaction was slowly reduced to room temperature within 1-2 hours, making the two phosphorylated single strand primers form a double strand DNA through base pairing. The resulting product was then diluted by 20-fold with ddH₂O. The double strand DNA obtained above was inserted into the BsaI site of plasmid pgRNA with the following reaction system: 1 µL of 10× T4 DNA ligase Buffer, 1 µL of the above 20-fold diluted phosphorylated double strand DNA, 20 fmol of plasmid pgRNA obtained in Example 2, 0.5 µL of T4 DNAligase (400 units/µL), 0.5 µL of BsaI-HF (20 units/µL), and finally appropriate amount of ddH₂O was added to a total volume of 10 µL. The buffer and enzyme used in this reaction were produced by NEB. The reaction was carried out in a PCR Amplifier under the following cycling conditions: 37°C for 2 min; 16°C for 5 min, under a total of 25 cycles; then 50°C for 5 min and 80°C for 15 min. 10 µL of the reaction product was transformed into *E. coli* competent DH5α strain and plated on LB solid culture plates containing 50 µg/mL of Kanamycin. After the transformation solution was absorbed by the solid culture plate, the plate was inverted and incubated overnight at 37°C in an incubator. The transformed strains grown on the plate were transferred and stored, and the plasmids were extracted and then sent to Sangon Biotech (Shanghai) Co., Ltd. for sequencing confirmation.

### Example 4 Preparation of Klebsiella pneumoniae electroporation-competent strain containing plasmid pCas12f

The *Klebsiella pneumoniae* strain was streaked on a LB solid culture plate, and the plate was inverted and incubated overnight at 37°C in an incubator. A monoclonal colony grown on the culture plate was picked and inoculated into 3 mL of LB liquid medium, and shaking overnight with 250 rpm at 37°C in a shaker. The next day, 1 mL of the bacterial liquid was inoculated into 100 mL of fresh LB medium and continue shaking at 37°C in a shaker. When the OD₆₀₀ of the bacterial liquid reached 0.6, the bacterial liquid was cooled on ice for ten minutes. The bacteria were collected by centrifugation at 6000 rpm for 5 minutes in a centrifuge at 4°C. The supernatant of the medium was discarded and the bacterial precipitation at the bottom was resuspended in 20 mL of 10% (v/v) glycerol (autoclaved and pre-cooled on ice). The resuspension was centrifuged at the same speed, the supernatant was discarded, and the bacterial precipitation at the bottom was resuspended again with 20 mL of 10% (v/v) glycerol. The resuspension was centrifuged at the same speed again, the supernatant was discarded, and the bacterial precipitation at the bottom was resuspended with 1 mL of 10% (v/v) glycerol. The resulting bacterial electroporation-competent strain was dispensed into EP tubes, with 50 µL of bacterial liquid per tube. The dispensed bacterial liquid was rapidly frozen in liquid nitrogen, following storation in a -80°C refrigerator. It should be noted that, the action should be gentle when resuspension, and the bacteria can be resuspended by gently blowing with a pipette.

A tube of freshly prepared *Klebsiella pneumoniae* electroporation-competent was placed on ice for 5-10 min, and then 1-2 µg of the plasmid pCas12f prepared in Example 1 was added. The well-mixed mixture was transfered into a 1 mm electrotransfer cup (Bio-Rad) and then electroshocked in a GenePulser Xcell electroshocker (Bio-Rad) at room temperature. The electroshock parameters were 1800 V, 200 S2, and 25 µF. After the electroshock, 1 mL of LB liquid medium was added immediately, and the well-mixed mixture was transferred into a clean EP tube, shaking for 1-2 hours at 37°C in a shaker. The EP tube was centrifuged at 8000 rpm for 1.5 min at room temperature. About 900 µL of supernatant was discarded, and the remaining bacterial liquid (about 150 µL) was mixed well and plated on a LB solid culture plate containing 50 µg/mL of Apramycin. After the bacterial solution was absorbed by the solid culture plate, the plate was inverted and incubated overnight at 37°C in an incubator, and only the bacteria successfully transferred with the plasmid were able to grow on the culture plates containing Apramycin. A monoclonal colony was picked and shaking overnight with 250 rpm at 37°C in a shaker in 3 mL of LB liquid medium containing 50 µg/mL Apramycin. The next day, 1 mL of the bacterial liquid was inoculated into 100 mL of fresh LB medium containing 50 µg/mL Apramycin and continue shaking at 37°C in a shaker. When the OD₆₀₀ of the bacterial liquid reached 0.2, 1 mL of 20% arabinose was added into the bacterial liquid. The bacterial liquid was continued shaking for 2 h, then was cooled on ice for ten minutes. The bacteria were collected by centrifugation at 6000 rpm for 5 minutes in a centrifuge at 4°C. The supernatant was discarded and the bacterial precipitation at the bottom was resuspended in 20 mL of 10% (v/v) glycerol (autoclaved and pre-cooled on ice). The resuspension was centrifuged again at the same speed, the supernatant was discarded, and the bacterial precipitation at the bottom was resuspended again with 20 mL of 10% (v/v) glycerol. The resuspension was centrifuged at the same speed again, the supernatant was discarded, and the bacterial precipitation was resuspended at the bottom with 1 mL of 10% (v/v) glycerol. The resulting bacterial electroporation-competent strain was dispensed into EP tubes, with 50 µL of bacterial liquid per tube. The dispensed bacterial liquid was rapidly frozen in liquid nitrogen firstly and then stored in a -80°C refrigerator. It should be noted that, the action should be gentle when resuspending the bacterial precipitation, and the bacteria can be resuspended by gently blowing with a pipette.

### Example 5 CRISPR- SpCas12f1 system for efficient gene deletion in living cells of Klebsiella pneumoniae

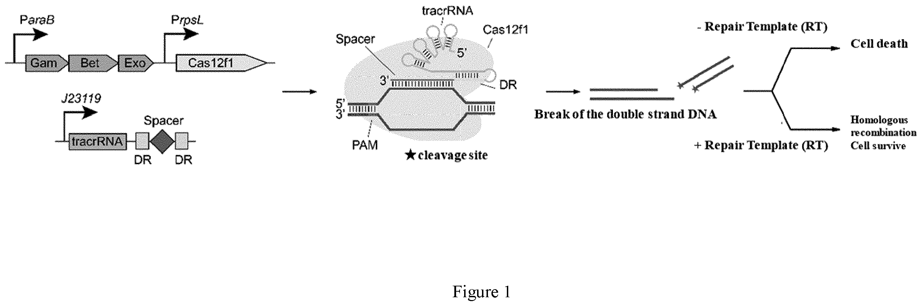

Efficient editing of different genes can be achieved in various cells using the CRISPR- SpCas12f1 system. A *Klebsiella pneumoniae* living cell was used as an example to show the method and process of knockout. The process is shown in Figure 1. Genes lacZ, dhaK and rpiR5 were selected as examples in the experiment. Figure 2a-d are graphs showing the results of gene knockout in living *Klebsiella* cells using the CRISPR-SpCas12f1 system. The results show that genes lacZ, dhaK and rpiR5 can be knocked out precisely by the CRISPR- SpCas12f1 system.

A tube of *Klebsiella pneumoniae* electroporation-competent strain prepared in Example 4 was placed on ice for 3-5 min, and after melting, 200 ng of plasmid pgRNA containing the spacer of the gene to be deleted and the homologous recombinant template of the gene to be deleted with a final concentration of 5 µM (total volume should not exceed 5 µL) was added and mixed gently. The mixed bacteria-plasmid mixture was transferred into a pre-cooled 1 mm electrotransfer cup (Bio-Rad) with a pipette, and standed on ice for 5 min. The condensation on the outer wall of the electrotransfer cup was wiped off, and the bacteria-plasmid mixture was subject to electroshock in a GenePulser Xcell electroshocker (Bio-Rad). The electroshock parameters were: 1800 V, 200 S2, and 25 µF, and the normal shock time is 4.8-5.3 ms. After the electroshock, 1 mL of LB culture solution was added immediately to wash out the electroshocked cells, and the cells were transferred into a sterile EP tube, and incubated to anabiosis at 37°C in a shaker with 200 rpm/min for 1 h. The anabiosis liquid was spread on LB solid medium containing Apramycin and Kanamycin, and after the solution was absorbed, the plate was inverted and incubated overnight at 37°C in an incubator.
Herein the repairing template sequence of gene lacZ is: 
5’-
TGTCGTTGCCCACCGTCAGCCGTTAGGCACCCCGACGGTGGATGATGTACC
ACTGGCTGAAGCGTAACGACCCGACCCGTCCGGTGCAGT-3’(SEQ ID NO: 25)
Herein the repairing template sequence of gene dhaK is: 
5’-
GAGCGACCCGGCGATCCGGGTGGTGGTCCGTCGCGACCTGAATAAGCGCG
CCGTCTCGGCTACAACGTGGAAATGCTGATCGTCGGCGAC-3’(SEQ ID NO:
26)
Herein the repairing template sequence of gene rpiR5 is:

5’-
CGTATTCGTCAGCGCTATCCCACCCTGGCGGCGAGCGACAAAAAGGATCTG
GAGCGGGCGCCGGAGCGCATTCGCCATAGCGAAGCGCTG-3’(SEQ ID NO: 27)

Colony PCR was performed on the grown colonies to verify the effect of the editing.
Herein the 5' primer of the colony PCR for gene lacZ knockout is:
   5'- ATGTGGCGGATGAGCGGTAT-3' (SEQ ID NO: 28)
Herein the 3' primer of the colony PCR for gene lacZ knockout is:
   5'- CCAGTAATCGGCGAAGTTGC -3'(SEQ ID NO: 29)
Herein the 5' primer of the colony PCR for gene dhaK knockout is:
   5'- CTTTGTCCGTTACCGCTCCG -3'(SEQ ID NO: 30)
Herein the 3' primer of the colony PCR for gene dhaK knockout is:
   5'- GGTTGACGATGTCCGCACTG -3'(SEQ ID NO: 31)
Herein the 5' primer of the colony PCR for gene rpiR5 knockout is:
   5'-CTGTTGATGCAAGGTTCCGTCC-3'(SEQ ID NO: 32)
Herein the 3' primer of the colony PCR for gene rpiR5 knockout is:
   5'-TTCTCGACGTGTGCCGGATC-3'(SEQ ID NO: 33)

### Example 6 CRISPR-SpCas12f1 system for efficient DNA double strand cleavage

The CRISPR-SpCas12f1 system can cleave double strand DNA efficiently. In this example, gene lacZ was used as an example to demonstrate the method and process of the cleavage.

The preparation of guide RNA. The preparation of the guide RNA was achieved by transcription *in vitro.* The transcription template DNA was synthesized by GENEWIZ Biotechnology Co., Ltd., the sequence of which is SEQ ID NO: 34:

5’-
TTAATACGACTCACTATAGGGaagcctgacataatttactctgtttcgcgcgccagggcagttaggtgccc
taaaagagcgaagtggccgaaaggaaaggctaacgcttctctaacgctacggcgaccttggcgaaatgccatcaatacca
cgcggcccgaaagggttcgcgcgaaactgagtaataaaacattgcggatgcggcaatacagaaccgctgtcgcatcttgc
gtaagcgcgtggattgaaacATGGCGGTTAACGCCGCGGA-3’

The guide RNA was prepared using the mentioned above template by HiScribe T7 High Yield RNA Synthesis Kit (New England Biolabs). The prepared guide RNA was purified by phenol-chloroform extraction and ethanol precipitation.

The preparation of SpCas12f1 nuclease. The SpCas12f1 gene was amplified by PCR amplification and cloned into *E. coli* expression vector pET28a. The successfully cloned plasmid was transferred into *E*. *coli* protein expression strain BL21 (DE3). The BL21 (DE3) strain containing the SpCas12f1 expression vector was cultured in 1 L LB at 37°C. When the OD₆₀₀ reached 0.6, 1 mL of 1 M IPTG was added to the culture medium and the temperature was lowered to 16°C to culture overnight. The strains cultured overnight were collected, sonicated, and purified using Ni-NTA (GE Healthcare) columns. The purified protein was further purified by Gel filtration (GE Healthcare). The purified protein was stored in a buffer containing 750 mM NaCl, 10 mM Tris-HCl, pH 7.5, and 1 mM DTT.

The preparation of cleavage template DNA gene lacZ. The cleavage template DNA gene lacZ was obtained by PCR amplification using *Klebsiella pneumoniae* genomic DNA as a template with gene lacZ-deleted colone PCR primers (SEQ ID NO: 28 and SEQ ID NO: 29). The PCR products were recovered separately using the SanPrep Column PCR Product Purification Kit manufactured by Sangon Biotech (Shanghai) Co., Ltd.

The *in vitro* cleavage experiments were conducted at conditions as 25 mM NaCl, 5 mM MgCl₂, 10 mM Tris-HCl, pH=7.5. The total reaction volume was 10 µL, including 10 nM gene lacZ fragment, 250 nM SpCas12f1, and 500 nM guide RNA. The reaction time was 30 min and reaction temperature was 37°C. At the end of the reaction, 25 mM of EDTA and 10 µg of protease K were added and then the reaction was terminated by heating at 58°C for 10 min. The reaction products were separated by 1% agarose gel and imaged by staining with 4S Red dye (Sangon Biotech (Shanghai) Co., Ltd.).

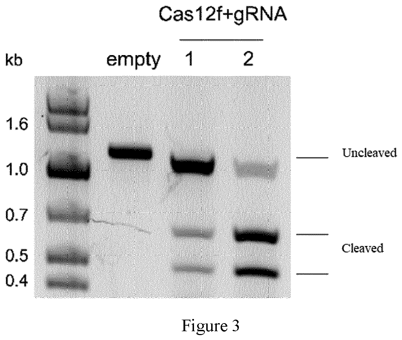

Figure 3 shows the result of the cleavage of the gene lacZ sequence using CRISPR- SpCas12f1 system. The results show that the gene lacZ amplified by PCR *in vitro* was precisely cut into two DNA fragments by CRISPR-SpCas12f1.

### Example 7 Construction of identification plasmids p15a-PAMdepletion and p15α-PAMcontrol of the protospacer adjacent motif (PAM) of AsCas12f1

The amino acid sequence of the AsCas12f1 nuclease described in this example is SEQ ID NO: 35. Preferably, the coding gene sequence of AsCas12f1 is SEQ ID NO: 36 after condon optimization according to *E*. *coli.* The backbone sequence of the guide RNA corresponding to the AsCas12f1 nuclease is SEQ ID NO: 37.

The sequence of plasmid p15a-PAMdepletion is SEQ ID NO: 38, and the plasmid was constructed as follows.

The p15a plasmid backbone (with the sequence as SEQ ID NO: 39), the *E*. *coli* codon optimized AsCas12f1 encoding gene expression cassette (with the sequence as SEQ ID NO: 40) and the guide RNA expression cassette corresponding to AsCas12f1 (with the sequence as SEQ ID NO: 41) were synthesized by Sangon Biotech (Shanghai) Co., Ltd. The three fragments were assembled into the plasmid p15a-PAMdepletion by Gibson assembly technology. The assembled plasmid was transformed into *E. coli* DH5α commercial competent cells, and were coated on the LBA plate with Apramycin. The monoclonal clones were selected for amplification culture, and the plasmids were extracted. After the insertion sequence was identified by sequencing, the plasmid p15a-PAMdepletion was obtained.

The sequence of plasmid p15a-PAMcontrol is SEQ ID NO: 42, and the plasmid was constructed as follows.

The p15a plasmid backbone (with the sequence as SEQ ID NO: 39), the *E*. *coli* codon optimized AsCas12f1 encoding gene expression cassette (with the sequence as SEQ ID NO: 40) and the control guide RNA expression cassette (with the sequence as SEQ ID NO: 43) were synthesized by Sangon Biotech (Shanghai) Co., Ltd. The three fragments were assembled into the plasmid p15a-PAMcontrol by Gibson assembly technology. The assembled plasmid was transformed into *E. coli* DH5α commercial competent cells, and were coated on the LBA plate with Apramycin. The monoclonal clones were selected for amplification culture, and the plasmids were extracted. After the insertion sequence was identified by sequencing, the plasmid p15a-PAMcontrol was obtained.

### Example 8 Construction of the protospacer adjacent motif (PAM) identification substrate plasmid pUC19-6N-library

The sequence of plasmid pUC19-6N-library is SEQ ID NO: 44, and the plasmid was constructed as follows.

The commercial plasmid pUC19 was used as the template for circular polymerase extension cloning using primer 6N-F (with the sequence is SEQ ID NO: 45) with 6N random sequence and common primer 6N-R (with the sequence as SEQ ID NO: 46). The PCR products were digested by Dpn1 and transformed into *E. coli* DH5α commercial competent cells, and coated on the LBA plate with Carbenicillin. More than 100,000 monoclonal clones were collected by scraping with a spreader, and mixed to extracted plasmids, and the protospacer adjacent motif (PAM) identification substrate plasmid pUC19-6N-library was obtained.

### Example 9 The protospacer adjacent motif (PAM) of AsCas12f1

The plasmid p15a-PAMdepletion and plasmid p15a-PAMcontrol were transformed into *E*. *coli* DH5α commercial competent cells by heat-shock respectively, and coated on the LBA plate with Apramycin. The monoclonal clones were taken into 100 mL of fresh LB medium the next day for incubation, and the bacterial cells were collected when OD₆₀₀ = 0.5. The bacteria were washed with 4°C sterilized water once, 4°C sterilized 10% glycerol twice, and finally the bacteria were resuspended with 1 mL sterilized 10% glycerol solution to obtain the competent cells. 100 ng of plasmid pUC19-6N-library was electrotransformed into DH5α competent cells with plasmid p15a-PAMdepletion and plasmid p15a-PAMcontrol respectively, and were coated on the LBA plate with Carbenicillin and Apramycin respectively. More than 100,000 monoclonal clones were collected by scraping with a spreader respectively, mixed to extract the plasmids, and the PAM depletion library and the control library were obtained. The first round of PCR was performed using PAM depletion library and control library as templates respectively and primers PAM-1F and PAM-1R (with the sequences as SEQ ID NO: 47 and No: 48), and the products were detected by electrophoresis. The second round of PCR was performed using 1 µL of the first round PCR products as template and primers PAM-2F and PAM-2R (with the sequences as SEQ ID NO: 49 and No: 50), and the products were detected by electrophoresis. The products were purified respectively using VAHTS DNA Clean Beads (Vazyme Biotech Co., Ltd.). High-throughput sequencing was performed on Illumina Hiseq2500 platform respectively, and 1 Gb of raw data was obtained for each. 6N random sequence information was extracted, and the sequence frequencies in the PAM depletion library and that in the control library were compared to establish the weblogo, thereby the information of the preferred protospacer adjacent motif (PAM) of AsCas12f1 was obtained.

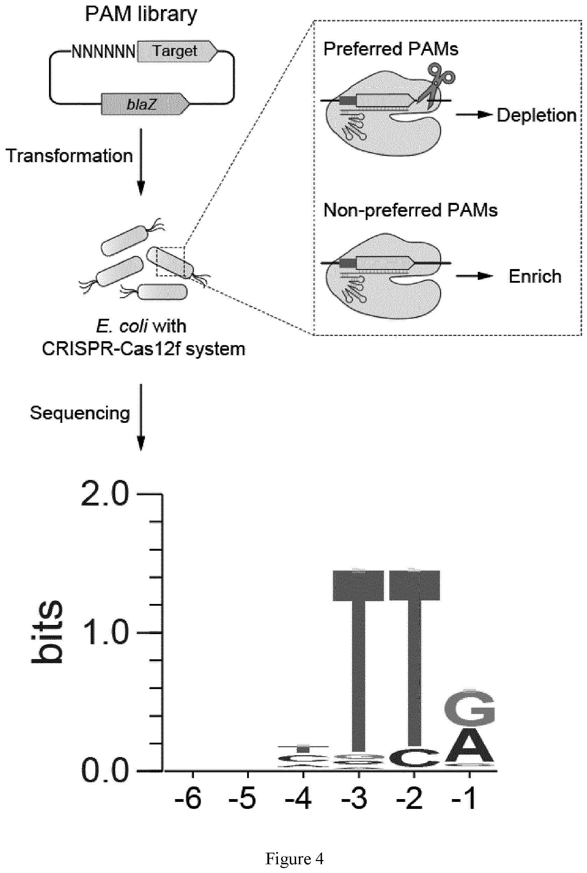

Figure 4 is the flow and results of the identification of the protospacer adjacent motif (PAM) of AsCas12f1. The results show that AsCas12f1 can recognize PAM sequences of 5'-TTR type effectively (wherein R is a degenerate base, representing A or G).

### Example 10 Construction of protein heterologous expression plasmid pET28a-SUMO- AsCas12f1 of AsCas12f1

The sequence of plasmid pET28a-SUMO-AsCas12f1 is SEQ ID NO: 51, the plasmid was constructed as follows:

The commercial plasmid pET28a was used as the template for PCR amplification using primer 28a-F (with the sequence as SEQ ID NO: 52) and primer 28a-R (with the sequence as SEQ ID NO: 53). The PCR products were digested by Dpn1 and then recovered by gel. The encoding gene sequence of AsCas12f1 with SUMO tag (SEQ ID NO: 54) was synthesized by Sangon Biotech (Shanghai) Co., Ltd. The two fragments were assembled into plasmid pET28a-SLTMO-AsCas12f1 by Gibson assembly technology. The assembled plasmid was transformed into *E. coli* DH5α commercial competent cells, then coated on the LBA plate with Kanamycin. The monoclonal clones were selected for amplification culture to extract the plasmids. After the insertion sequence was identified by sequencing, the plasmid pET28a-SUMO-AsCas12f1 was obtained.

### Example 11 AsCas12f1 achieves efficient double strand DNA cleavage in vitro

The preparation of the complete guide RNA targeting linearized plasmid pUC19. The preparation of the complete guide RNA was achieved by transcription *in vitro.* The transcriptional template DNA was synthesized by GENEWIZ Biotechnology Co., Ltd., with the sequence of which is SEQ ID NO: 55.

The complete guide RNA was prepared using the mentioned above template by HiScribe T7 High Yield RNA Synthesis Kit (New England Biolabs). The prepared complete guide RNA was purified by phenol-chloroform extraction and ethanol precipitation.

The preparation of AsCas12f1 nuclease. The plasmid pET28a-SUMO-AsCas12f1 was transformed into *E. coli* protein expression strain BL21 (DE3). The next day, the transformants were transferred to 1 L LB medium for shaking culture at 37°C. When the OD₆₀₀ reached 0.6, 0.25 mL of 1 M IPTG was added to the culture medium and the temperature was lowered to 16°C for culture overnight. The strains cultured overnight were collected, sonicated, and purified using Ni-NTA (GE Healthcare) columns. The purified protein was tag-removed with HRV3c protease and further purified by HiLoad 16/600 Superdex 200pg molecular sieve (GE Healthcare). The purified protein was stored in a buffer containing 1000 mM NaCl, 10 mM Tris-HCl, pH 7.5, and 1 mM DTT.

The preparation of cleavage substrate. The cleavage substrate was linearized plasmid pUC19. The commercial plasmid pUC19 was used as the template, and the primers UC-F and UC-R (SEQ ID NO: 56 and SEQ ID NO: 57) were used to amplify the whole plasmid. After the PCR product was recovered by gel, the linearized cleavage substrate was obtained.

The *in vitro* cleavage experiments were conducted at conditions as: 50 mM NaCl, 10 mM MgCl₂, 10 mM Tris- HCl, and pH=7.5. The total reaction volume was 20 µL, including 5 nM of the cleavage substrate, 250 nM of AsCas12f1, and 250 nM of the guide RNA. The reaction time was 30 min and reaction temperature was 45°C. At the end of the reaction, 50 mM of EDTA was added to terminate the reaction. The reaction products were separated by 1% agarose gel and imaged by staining with 4S Red dye (Sangon Biotech (Shanghai) Co., Ltd.).

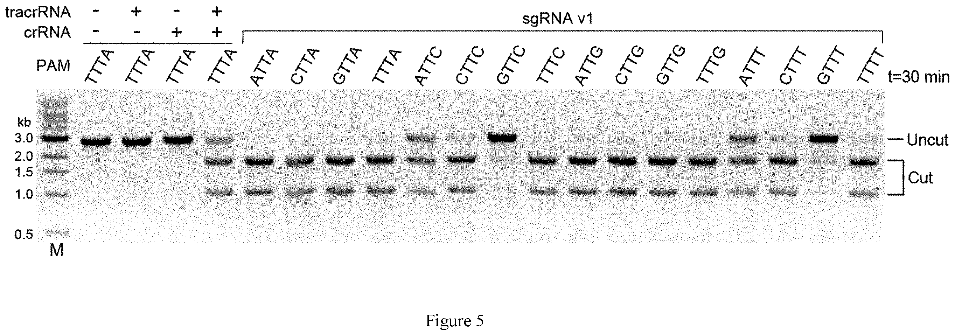

Figure 5 is the results of cleavage of substrate DNA by AsCas12f1 nuclease. The results show that the substrate DNA was cut into two DNA fragments by AsCas12f1 precisely. Its cleavage activity depends on the recognition of the PAM sequence, and the regularity is fully consistent with the protospacer adjacent motif (PAM) obtained from the identification.

### Example 12 The break types of double strand DNA cleaved by AsCas12f1

The preparation of the complete guide RNA targeting short fluorescence labeled synthetic DNA was the same as described in Example 11.

The preparation of AsCas12f1 nuclease was the same as described in the Example 11.

The preparation of cleavage substrate. The cleavage substrates were four fluorescence labeled synthetic DNA sequences with a length of 56 bp (synthesized by Sangon Biotech (Shanghai) Co., Ltd.), the sequence of which is SEQ ID NO: 58. The FAM fluorescent moieties were labeled individually at the two 5' terminuses and two 3' terminuses, respectively.

The *in vitro* cleavage experiments were conducted at conditions as: 50 mM NaCl, 10 mM MgCl₂, 10 mM Tris- HCl, and pH=7.5. The total reaction volume was 40 µL, including 20 nM of the cleavage substrate, 400 nM of AsCas12f1, and 400 nM of guide RNA. The reaction time was 30 min and reaction temperature was 45°C. 10 µL of samples were collected at 5, 10, 20 and 30 min, respectively, and the reaction was terminated by adding 2×formamide loading buffer (Sangon Biotech (Shanghai) Co., Ltd.). The reaction products were separated by 20% TBE-Urea-PAGE and imaged by excitation of 488 nm blue light.

Figure 6 is the results of cleavage of four fluorescence labeled DNA with a length of 56 bp substrates with AsCas12f1 nuclease. The results show that the cleavage site of AsCas12f1 in the targeting strand is 3-4 nt downstream the sequence complementary to the spacer, while the cleavage site in the non-targeting strand is between the positions 12 and 13 in the protospacer. After the specific cleavage of the non-targeting strand, AsCas12f1 can also non-specifically cleave the single strand DNA of the non-targeting strand replaced by the binding of sgRNA, with the cleavage length concentrated at about 13 nt. The resulting cleavage product has a sticky protruding end with a length of 11 nt at 5' terminus, and a sticky protruding end with a length of 0-4 nt at 3' terminus.

### Example 13 Preferred biochemical reaction conditions for AsCas12f1

The preparation of the complete guide RNA targeting the linearized plasmid pUC19 was the same as described in the Example 11.

The preparation of AsCas12f1 nuclease was the same as described in the Example 11.

To determine the optimal reaction conditions for AsCas12f1 in cleavage *in vitro,* the preference of AsCas12f1 for the types and concentrations of divalent cations, concentration of NaCl, temperature of the reaction, and length of Spacer were studied respectively. The total reaction time was 40 min. Samples were collected at 2, 5, 10, 20 and 40 min, respectively. The reaction products were separated by 1% agarose gel and imaged by staining with 4S Red dye (Sangon Biotech (Shanghai) Co., Ltd.). The cleavage efficiency at each time point was obtained by grayscale analysis of the electropherograms.

Figure 7a and Figure 7b are the results of the preference of AsCas12f1 nuclease for the types and concentrations of divalent cations. The results show that the activity of AsCas12f1 depends on Mg²⁺, and its concentration should be 5 mM or more. Figure 7c is the results of the preference of AsCas12f1 nuclease for NaCl concentration. The results show that AsCas12f1 is relatively sensitive to NaCl concentration, and its concentration should be 100 mM or less. Figure 7d is the results of the temperature preference of AsCas12f1 nuclease. The results show that the optimum temperature for cleavage of DNA by AsCas12f1 is 45-60 centigrade.

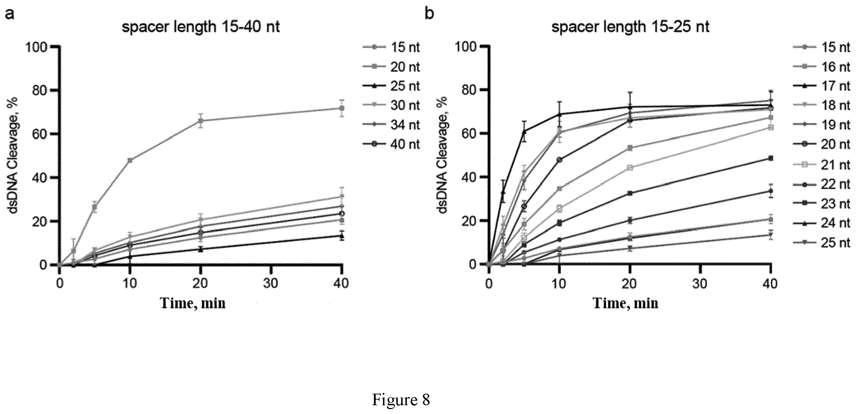

Figure 8a and Figure 8b are the results of the preference of AsCas12f1 nuclease for spacer length. The results show that the optimal spacer length of AsCas12f1 is between 17-20 nt.

### Example 14 Key amino acid site for double strand DNA cleavage activity of AsCas12f1

The following four key amino acid residues associated with DNA cleavage activity were identified by structural modeling of the protein AsCas12f1: D225, E324, R383 andD401. The plasmid pET28a-SUMO-AsCas12f1 was used as a template and point mutation primers targeting each of these four amino acid residues (with the sequences as SEQ ID NO: 59-66) for cyclic polymerase extension cloning. Four heterologous expression plasmids of protein AsCas12f1with amino acid point mutations were obtained, pET28a-SUMO-AsCas12f1-D225A, pET28a-SUMO-AsCas12f1-E324A, pET28a-SLTMO-AsCas12f1-R383A and pET28a-SUMO-AsCas12f1-D401A (the sequences are SEQ ID NO: 67-70, respectively).

The preparation of the complete guide RNA, AsCas12f1 nuclease and the point mutants thereof were as described in Example 11. The identification of the cleavage activity of the mutants was performed as described in Example 12.

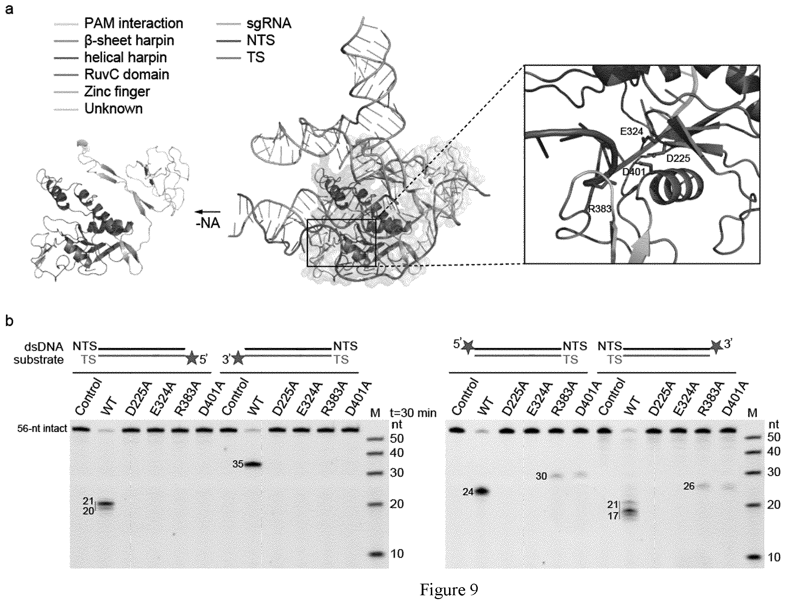

Figure 9a is the results of structural simulation of AsCas12f1. Figure 9b is the results of double strand DNA cleavage activity of the four mutants compared with wild-type AsCas12f1 protein. The results show that all four mutants inactivated the cleavage ability of AsCas12f1 on the targeting strand, while mutants R383A and D401A still retained weak cleavage activity on the non-targeting strand (nickase activity).

### Example 15 Construction of bacterial in vivo expression plasmid p15a-AsCas12f1 of AsCas12f1

The sequence of plasmid p15a-AsCas12f1 is SEQ ID NO: 71, and the plasmid was constructed as follows.

The p15a plasmid backbone (with the sequence as SEQ ID NO: 39), the AsCas12f1 encoding gene expression cassette after *E. coli* codon optimization (with the sequence as SEQ ID NO: 40) and the lambda-red recombinase expression cassette (with the sequence as SEQ ID NO: 72) were synthesized by Sangon Biotech (Shanghai) Co., Ltd. The three fragments were assembled into the plasmid p15a-AsCas12f1 by Gibson assembly technology. The assembled plasmid was transformed into *E. coli* DH5α commercial competent cells, and were coated on the LBA plate with Apramycin. The monoclonal clones were selected for amplification culture to extract the plasmids. After the insertion sequence was identified by sequencing, the plasmid p15a-AsCas12f1 was obtained.

### Example 16 Construction of the guide RNA expression plasmid psgRNAv1 corresponding to AsCas12f1

The sequence of plasmid psgRNAv1 is SEQ ID NO: 73, and the plasmid was constructed as follows.

The plasmid backbone was obtained by using the commercial plasmid pSGKP as a template and using the primers SGKP-F and SGKP-R (with the sequences as SEQ ID No: 74 and 75), and the guide RNA expression cassette containing two Bsa1 sites (with the sequence as SEQ ID NO: 76) was synthesized by Sangon Biotech (Shanghai) Co., Ltd. The two fragments were assembled into the plasmid psgRNAv1 by Gibson assembly technology. The assembled plasmid was transformed into *E. coli* DH5α commercial competent cells, and were coated on the LBA plate with Kanamycin. The monoclonal clones were selected for amplification culture to extract the plasmids. After the insertion sequence was identified by sequencing, the plasmid psgRNAv1 was obtained.

### Example 17 AsCas12f1 for efficient gene editing in living cells of E. coli and Klebsiella pneumoniae

The targeting sequence DNA was inserted into the plasmid psgRNAv1. Gene hisD of *E. coli* and gene pyrF of *Klebsiella pneumoniae* were selected as target sequences in this example, and 20 bp of targeting sequence DNA was selected on the target sequence, respectively. Oligonucleotide sequences hisD-F, hisD-R, pyrF-F and pyrF-R were synthesized respectively (with the sequences as SEQ ID NO: 77-80). The hisD-F and hisD-R, and pyrF-F and pyrF-R were mixed in 1×T4 DNAligase buffer, respectively, then 1 mM ATP and 1 µL of T4 PNK was added, and the mixture was reacted for 1 h at 37°C. After that, 50 mM NaCl was added additionally and the reaction was annealed slowly. Subsequently, two targeting sequences DNA were inserted into plasmid psgRNAv1 respectively by Golden gate assembly technology, and plasmids psgRNAv1-hisD and psgRNAv1-pyrF were constructed respectively (with the sequences as SEQ ID NO: 81 and 82).

*E. coli* or *Klebsiella pneumoniae* were streaked on LBA plates, inverted and incubated overnight at 37°C in an incubator for activation. Monoclonal colonies were picked and inoculated into 100 mL of fresh LB medium, and continued shaking at 37°C in a shaker. When the OD₆₀₀ of the bacterial liquid reached 0.6, the bacteria were collected by centrifugation, washed with 4°C sterilized water once, 4°C sterilized 10% glycerol twice, and finally the bacteria were resuspended with 1 mL sterilized 10% glycerol solution to obtain the electroporation-competent cells. 100 ng of the plasmid p15α-AsCas12f1 was electrotransformed into the fresh electroporation-competent cells, and the cells were coated on the LBA plate with Apramycin, inverted and incubated overnight at 37°C in an incubator. The transformants were picked the next day and inoculated into 100 mL of fresh LB medium containing 0.2% arabinose, and continued shaking at 37°C in a shaker. When the OD₆₀₀ of the bacterial solution reached 0.6, the bacteria were collected by centrifugation, washed with 4°C sterilized water once, 4°C sterilized 10% glycerol twice, and finally the bacteria were resuspended with 1 mL sterilized 10% glycerol solution to obtain the electroporation-competent cells. 100 ng of psgRNAv1 plasmid targeting different target sequences and 1 µg of synthetic single strand repair template targeting different target sites (with the sequences as SEQ ID NO: 83 and 84) were electrotransformed into the fresh electroporation-competent cells, and coated on the LBA plate with Carbenicillin and Apramycin, inverted and incubated overnight at 37°C in an incubator. The next day, monoclonal colonies were picked for PCR and sequenced to identify the gene editing results.

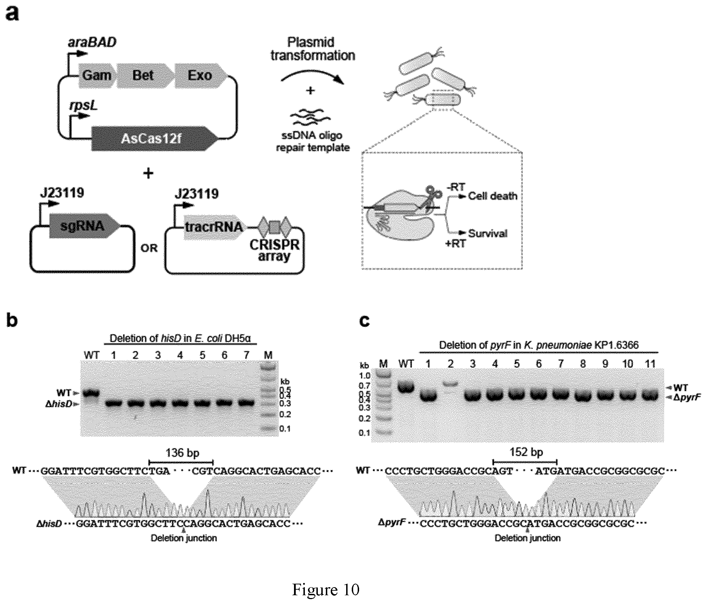

The AsCas12f1 nuclease enables efficient editing of different genes in a variety of cells. This example shows gene editing methods and processes in living cells of *E*. *coli* and *Klebsiella pneumoniae* for examples. The process is shown in Figure 10a. The hisD gene in *E. coli* and the pyrF gene in *K. pneumoniae* were selected for the experiments, respectively. Figure 10b is the results of gene editing in *E. coli* using AsCas12f1 nuclease. Figure 10c is the results of gene editing in *K. pneumoniae* using AsCas12f1 nuclease. The results show that the hisD gene in *E*. *coli* and the pyrF gene in *K. pneumoniae* can be precisely edited by AsCas12f1 nuclease.

### Example 18 Construction of mammalian cell gene editing plasmid pAsCas12fHs of AsCas12f1

The AsCas12f1 described in this example uses a preferred AsCas12f1 encoding gene with condon optimization for human, which has the sequence of SEQ ID NO: 85.

The sequence of plasmid pAsCas12fHs is SEQ ID NO: 86, and the plasmid was constructed as follows.

The human transient expression plasmid backbone (with the sequences as SEQ ID No: 87), the puromycin resistance gene expression cassette sequence (with the sequence as SEQ ID NO: 88), the expression cassette sequence of AsCas12f1 encoding gene with condon optimization for human (with the sequence as SEQ ID NO: 89) and the guide RNA expression cassette corresponding to AsCas12f1 in human cells (with the sequence as SEQ ID NO: 90) were synthesized by Sangon Biotech (Shanghai) Co., Ltd. The four fragments were assembled into the plasmid pAsCas12fHs by Gibson assembly technology. The assembled plasmid was transformed into *E. coli* DH5α commercial competent cells, and were coated on the LBA plate with Carbenicillin. The monoclonal clones were selected for amplification culture to extract plasmids. After the insertion sequence was identified by sequencing, the plasmid pAsCas12fHs was obtained.

### Example 19 Construction of protein heterologous expression plasmid pET28a-SUMO-AsCas12f1-2NLS for AsCas12f1

The sequence of plasmid pET28a-SLTMO-AsCas12f1-2NLS is SEQ ID NO: 91, and the plasmid was constructed as follows.

The commercial plasmid pET28a was used as the template for PCR amplification using primer 28a-F (with the sequence as SEQ ID NO: 52) and primer 28a-R (with the sequence as SEQ ID NO: 53). The PCR products were digested by Dpn1 and then recovered by gel. The encoding gene sequence of AsCas12f1 with SUMO tag+nuclear localization signal at the N-terminus and C-terminus respectively (with the sequence as SEQ ID NO: 92) was synthesized by Sangon Biotech (Shanghai) Co., Ltd. The three fragments were assembled into plasmid pET28a-SUMO-AsCas12f1-2NLS by Gibson assembly technology. The assembled plasmid was transformed into *E. coli* DH5α commercial competent cells, and were coated on the LBA plate with Kanamycin. The monoclonal clones were selected for amplification culture to extract plasmids. After the insertion sequence was identified by sequencing, the plasmid pET28a-SUMO-AsCas12f1-2NLS was obtained.

### Example 20 Efficient gene editing in living cells of human by AsCas12f1

Efficient gene editing in human living cells can be achieved using AsCas12f1 nuclease. Human embryonic kidney cell HEK293 was used as a model to demonstrate the effect of gene editing of AsCas12f1.

The targeting sequences DNA were inserted into the plasmid pAsCas12fHs. In this example, five genes VEGFA, TP53, HEXA, PRNP, and PDCD1 in the human genome were selected as target sequences, and seven 20-bp targeting sequences DNA were selected from these target sequences, respectively. Oligonucleotide sequences Guide1-F, Guide1-R, Guide2-F, Guide2-R, Guide3-F, Guide3-R, Guide4-F, Guide4-R, Guide5-F, Guide5-R, Guide6-F, Guide6-R, Guide7-F and Guide7-R (with the sequences as SEQ ID NO: 93-106) were synthesized respectively. Guide1-F and Guide1-R, Guide2-F and Guide2-R, Guide3-F and Guide3-R, Guide4-F and Guide4-R, Guide5-F and Guide5-R, Guide6-F and Guide6-R, and Guide7-F and Guide7-R were mixed in 1×T4 DNA ligase buffer respectively, then 1 mM of ATP and 1 µL of T4 PNK was added, and the mixture was reacted for 1 h at 37°C. After that, 50 mM NaCl was added additionally and the reaction was annealed slowly. Subsequently, seven targeting sequences DNA were inserted into plasmid pAsCas12fHs respectively by Golden gate assembly technology, and plasmids pAsCas12fHs-1, pAsCas12fHs-2, pAsCas12fHs-3, pAsCas12fHs-4, pAsCas12fHs-5, pAsCas12fHs-6 and pAsCas 12fHs-7 were constructed respectively (with the sequences as SEQ ID NO: 107-113).

Gene editing mediated by transient expression plasmid in human cells:
The cryopreservated HEK293 cells were activated, and the HEK293 cells were subcultured into a 24-well plate with approximately 1.5×10⁵ cells per well after two days. 16-18 hours later, 500 ng of plasmids pAsCas12fHs editing different genes were transfected into the cells respectively using 0.75 µL of lipofectamine 3000 (Invitrogen), and the cells were continued to culture for 72 hours. Then the adherent cells were digested and their genomic DNA was extracted. The gene fragments were amplified by PCR with sequencing primers corresponding to the genes. The PCR products were recovered by gel. The PCR products were annealed with NEBuffer2 (NEB). Then T7 endonuclease 1(NEB) was added and enzyme digestion was performed at 37°C for 15 min. The reaction was terminated by adding 50 mM of EDTA, and the reaction products were separated by 6% TBE-PAGE and imaged by staining with 4S Red dye (Sangon Biotech (Shanghai) Co., Ltd.).

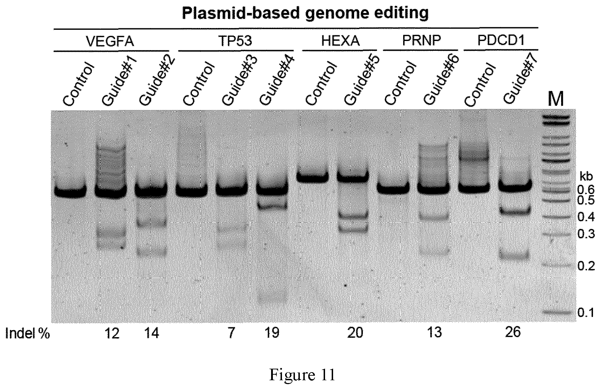

Figure 11 is the results of AsCas12f1 transient expression plasmid-mediated gene editing in human cells. As shown in the figure, AsCas12f1 nuclease successfully achieved efficient gene editing of seven sites on genes VEGFA, TP53, HEXA, PRNP and PDCD1.

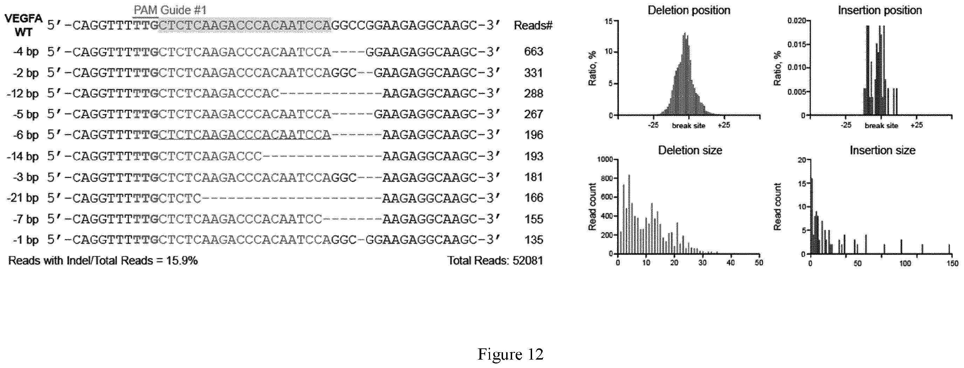

Figure 12 is the statistical results of high-throughput sequencing after AsCas12f1 transient expression plasmid-mediated gene editing in human cells. As shown in the figure, AsCas12f1 nuclease can introduce insertion or deletion mutations at the target sequence precisely.

Gene editing mediated by Ribonucleoprotein complex in human cells.

The sequences of the complete guide RNAs used in this example are SEQ ID NO: 114-119. The preparation of the complete guide RNA was the same as described in the Example 11.

The preparation of AsCas12f1-2NLS nuclease was the same as described in the Example 11.

The cryopreservated HEK293 cells were activated, and the HEK293 cells were subcultured into a 24-well plate with approximately 1.5×10⁵ cells per well two days later. 16-18 hours later, 300 pmole of AsCas12f1-2NLS and 300 pmole of the guide RNA for editing different genes were pre-mixed in 100 µL of Opti-MEM, an additional 10 mM Mg²⁺ was added, and incubated at 45°C for 10 min. Then 3 µL of lipofectamine 3000 (Invitrogen) was mixed and diluted in 100µL of Opti-MEM, and the mixed solutions were left at room temperature for 15 min and transfected into the cells respectively. The cells were cultured for another 48 hours. Then the adherent cells were digested and their genomic DNA was extracted. The gene fragments were amplified by PCR with sequencing primers corresponding to the genes. The PCR products were recovered by gel. The PCR products were annealed with NEBuffer2 (NEB). Then T7 endonuclease 1(NEB) was added and the enzyme digestion was performed at 37°C for 15 min. The reaction was terminated by adding 50 mM of EDTA, and then the reaction products were separated by 6% TBE-PAGE and imaged by staining with 4S Red dye (Sangon Biotech (Shanghai) Co., Ltd.).

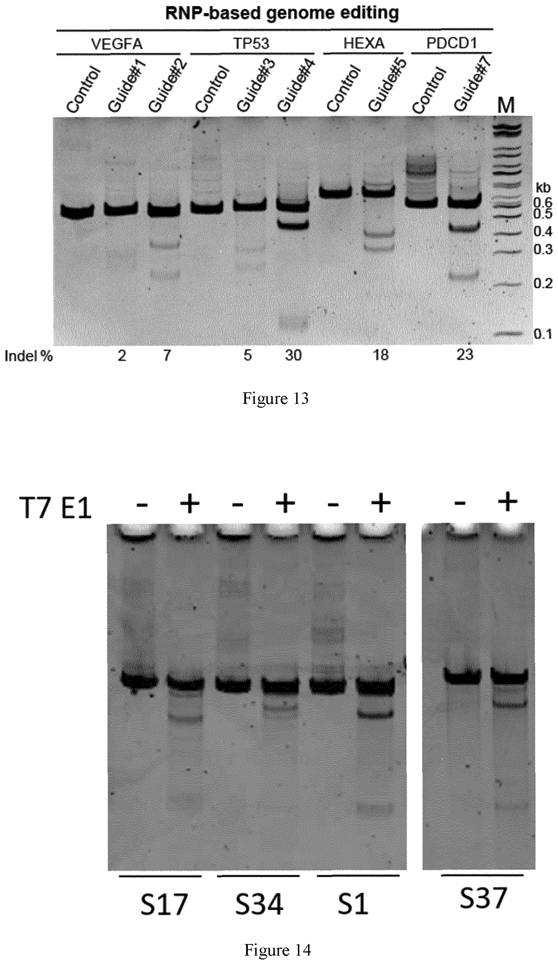

Figure 13 is the results of AsCas12f1 ribonucleoprotein complex-mediated gene editing in human cells. As shown in the figure, AsCas12f1 nuclease successfully achieved efficient gene editing of six sites on genes VEGFA, TP53, HEXA and PDCD1.

### Example 21 Efficient gene editing in living cells of human by SpCas12f1

The preferred encoding gene sequence of SpCas12f1 with condon optimization for human described in this example is SEQ ID NO: 120.

The mammalian cell gene editing plasmid pSpCas12fHs of SpCas12f1 was constructed as described in Example 18, and the sequence of which is SEQ ID NO: 121.

Efficient gene editing in living human cells can be achieved using SpCas12f1 nuclease. Human embryonic kidney cell HEK293 was used as a model to demonstrate the effect of gene editing of SpCas12f1.

The targeting sequences DNA were inserted into the plasmid pSpCas12fHs. In this example, the VEGFA gene in human genome was selected as the target sequence, four 20-bp targeting sequences DNA were selected respectively from the target sequence, and the plasmids pSpCas12fHs-S1, pSpCas12fHs-S17, pSpCas12fHs-S34 and pSpCas12fHs-S37 were constructed respectively (with the sequences as SEQ ID NO: 122-125).

Figure 14 is the results of SpCas12f1 transient expression plasmid-mediated gene editing in human cells. As shown in the figure, SpCas12f1 nuclease successfully achieved efficient gene editing of four sites on the gene VEGFA.

### Example 22 EsCas12f1 achieves efficient double strand DNA cleavage in vitro

The amino acid sequence of EsCas12f1 is SEQ ID NO: 126. Its preferred encoding gene sequence with condon optimization for *E*. *coli* is SEQ ID NO: 127. The method for constructing the protein heterologous expression plasmid pET28A-SUMO-EsCas12f1 (with the sequence as SEQ ID NO: 128) of EsCas12f1 was the same as described in Example 10.

The sequence of the guide RNA backbone corresponding to the EsCas12f1 is SEQ ID NO: 129.

The preparation of the complete guide RNA targeting the linearized plasmid pUC19 corresponding to the EsCas12f1 was the same as described in the Example 11, and the sequence of which is SEQ ID NO: 130.

The EsCas12f1 nuclease was prepared as described in Example 11.

The cleavage substrate was prepared as described in Example 11.

The cleavage experiments *in vitro* were performed as described in Example 11.

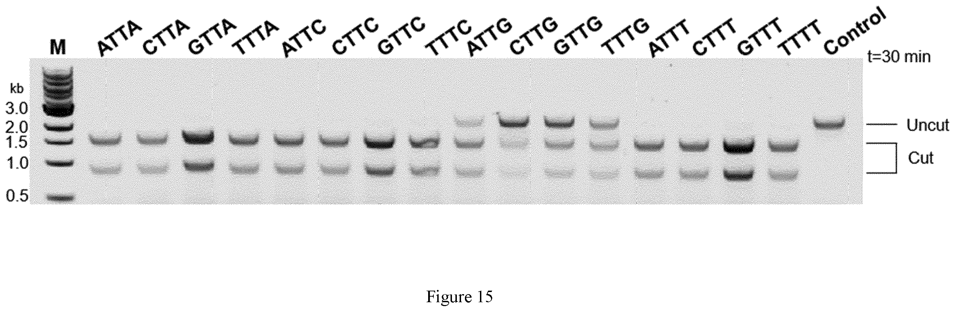

Figure 15 is the results of cleavage of substrate DNA by EsCas12f1 nuclease. The results show that the substrate DNA was cut into two DNA fragments by EsCas12f1 precisely, and EsCas12f1 preferred the 5'PAM sequence of 5'-TTH type (where H stands for A, C and T).

Although the above describes specific embodiments of the present dislosure, it should be understood by a person skilled in the art that these are merely illustrative examples and that a variety of changes or modifications can be made to these embodiments without departing from the principles and substance of the present disclosure. Therefore, the scope of protection of the present disclosure is limited by the appended claims.

## Claims

1. A genome editing system for gene editing at least one target sequence in a living cell genome, wherein the genome editing system comprising:
(1) an expression construct comprising a Cas12f nuclease;
(2) an expression construct comprising an expression DNA sequence of a guide RNA corresponding to the Cas12f nuclease, and a targeting sequence of the target sequence.

2. The genome editing system of claim 1, wherein the guide RNA is set forth in a sequence of SEQ ID NO: 3 in sequence listing;
and/or, the targeting sequence is a DNA fragment with a length of 20 bp downstream of PAM sequence.

3. The genome editing system of claim 1, wherein the Cas12f nuclease has a source selected from a group consisting of: *Syntrophomonas palmitatica* Cas12f (SpCas12f1), *Acidibacillus sulfuroxidans* Cas12f (AsCas12f1), *Eubacterium siraeum* Cas12f (EsCas12f1) and *Clostridium novyi* Cas12f (CnCas12f1).

4. The genome editing system of any one of claims 1 to 3, wherein the Cas12f nuclease is set forth in an amino acid sequence of SEQ ID NO: 1 in sequence listing; preferably, the Cas12f nuclease is set forth in a coding sequence of SEQ ID NO: 2 in sequence listing.

5. A genome editing method comprising introducing the genome editing system of any one of claims 1 to 4 into a cell comprising a target sequence to perform genome editing.

6. The genome editing system of any one of claims 1 to 4 or the genome editing method of claim 5, wherein the gene editing is selected from a group consisting of: gene cleavage, gene deletion, gene insertion, point mutation, transcription inhibition, transcription activation and base editing; preferably gene deletion or gene cleavage.

7. A cleavage method of double strand DNA comprising mixing Cas12f nuclease, complete guide RNA and the double strand DNA; wherein the complete guide RNA is: 5'- guide RNA corresponding to the Cas12f nuclease + targeting sequence of the double strand DNA-3'.

8. The cleavage method of claim 7, wherein the guide RNA corresponding to the Cas12f nuclease is shown in SEQ ID NO: 3 of sequence listing;
and/or, the Cas12f nuclease has a source selected from a group consisting of: *Syntrophomonas palmitatica* Cas12f (SpCas12f1), *Acidibacillus sulfuroxidans* Cas12f (AsCas12f1), *Eubacterium siraeum* Cas12f (EsCas12f1) and *Clostridium novyi* Cas12f (CnCas12f1); preferably the Cas12f nuclease is shown in SEQ ID NO: 1 of sequence listing; more preferably, the Cas12f nuclease is set forth in a coding sequence of SEQ ID NO: 2 in sequence listing;
and/or, the targeting sequence is a DNA fragment with a length of 20 bp downstream of PAM sequence.

9. A use of Cas 12f nuclease in gene editing;
preferably, the Cas12f nuclease has a source selected from a group consisting of: *Syntrophomonas palmitatica* Cas12f (SpCas12f1), *Acidibacillus sulfuroxidans* Cas12f (AsCas12f1), *Eubacterium siraeum* Cas12f (EsCas12f1) and *Clostridium novyi* Cas12f (CnCas12f1), etc.; the Cas12f nuclease is preferably set forth in an amino acid sequence of SEQ ID NO: 1 in sequence listing; more preferably the Cas12f nuclease is encoded by a nucleotide sequenceof SEQ ID NO: 2 in sequence listing.

10. The use of claim 9, wherein the gene editing is selected from a group consisting of: gene cleavage, gene deletion, gene insertion, point mutation, transcription inhibition, transcription activation and base editing; preferably gene deletion or gene cleavage.
